# EUROPÄISCHE PATENTSCHRIFT

(11) **EP 2 987 469 B1**
(45) Veröffentlichungstag und Bekanntmachung des Hinweises auf die Patenterteilung: **26.12.2018**
(21) Anmeldenummer: 15002132.7
(22) Anmeldetag: 23.03.2012
(51) Int. Cl.: A61F 2/72, A61F 2/78

(54) **EINRICHTUNG MIT EINER ZUM ENGEN UMSCHLIESSEN EINES KÖRPERTEILS AUSGEBILDETEN WANDUNG**
DEVICE WITH A WALL FOR TIGHTLY ENCLOSING A BODY PART
DISPOSITIF AVEC UNE PAROI CONÇUE POUR L'ENVELOPPEMENT ÉTROIT D'UNE PARTIE D'UN CORPS

(30) Priorität: 20.07.2011 DE 102011108136
(43) Veröffentlichungstag der Anmeldung: 24.02.2016
(62) Teilanmeldung aus: 12711780.2
(73) Patentinhaber: Ottobock SE & Co. KGaA, 37115 Duderstadt (DE)
(72) Erfinder: Mosler, Lüder, 37115 Duderstadt (DE); Graimann, Bernhard, 37434 Obernfeld (DE)
(74) Vertreter: Lins, Edgar

(56) Entgegenhaltungen:
- DE-A1-102007 035 409
- US-A- 5 443 525
- US-A1- 2009 216 339
- US-A1- 2010 114 238

## Beschreibung

Die Erfindung betrifft eine Einrichtung mit einer zum engen Umschließen eines Körperteils ausgebildeten Wandung mit einem elektrisch nicht leitenden Material und einer an das Körperteil angepassten oder sich aufgrund der Elastizität der Wandung anpassenden Form, wobei die Wandung mit einer Innenwand an der Haut des Körperteils zur Anlage kommt und die Innenwand mit wenigstens einem elektrisch leitenden Abschnitt versehen ist, der zur Übertragung elektrischer Signale von oder zu der Haut des Körperteils durch das elektrisch nicht leitende Material der Wandung hindurch angeordnet ist, wobei der elektrisch leitende Abschnitt von dem elektrisch nicht leitenden Material abgedeckt und mit wenigstens einem durch das elektrisch nicht leitende Material hindurchführenden Leiter verbunden ist.

Einrichtungen der hier angesprochenen Art sind eng um das Körperteil gewickelte Bandagen oder Liner, die über einen Amputationsstumpf gezogen werden. Die Liner weisen eine gewisse Wandstärke auf und haben die Funktion, eine polsternde und sich an den Amputationsstumpf anpassende oder angepasste Zwischenschicht zwischen dem Amputationsstumpf und der Innenwand eines Prothesenschafts auszubilden. Der Prothesenschaft ist Teil einer Prothese, die den amputierten Teil einer Extremität des Patienten ersetzt.

Die Übertragung elektrischer Signale zwischen dem Amputationsstumpf und der Außenseite des Liners kann aus mehreren Gründen in Betracht kommen. So kann es sinnvoll sein, elektrische Signale von der Haut des Amputationsstumpfes nach außen zu übertragen, um die Funktion der Prothese zu steuern. In diesem Fall können die Elektroden myoelektrische Elektroden sein, die an geeigneten Punkten des Amputations-stumpfes Muskelkontraktionssignale abnehmen, wodurch die Steuerung entsprechender Prothesenglieder erfolgen kann. Eine myoelektrische Steuerung von Prothesen ist insbesondere für Arm- und Handprothesen bekannt, kann aber auch für Bein- und Fußprothesen verwendet werden.

Ferner kann es sinnvoll sein, den Oberflächenwiderstand der Haut des Amputationsstumpfes elektrisch zu bestimmen, indem ein Stromfluss zwischen zwei oder mehreren Elektroden bzw. Elektrodenabschnitten gemessen wird. Auf diese Weise kann beispielsweise bestimmt werden, ob die Haut des Amputationsstumpfes innerhalb des Liners transpiriert, wodurch sich der Sitz des Liners auf dem Amputationsstumpf - und damit der Sitz der Prothese - verschlechtern kann. Ferner ist es möglich, mit Elektroden den Anpressdruck des Amputationsstumpfes an der Innenwand des Liners zu bestimmen, um so beispielsweise auf einen Masseschwund des Amputationsstumpfes während des Tragens der Prothese reagieren zu können.

Umgekehrt kann es sinnvoll sein, von der Außenseite des Liners auf die Haut des Amputationsstumpfes elektrische Signale zu übertragen, beispielsweise um eine Muskelkontraktion des Amputationsstumpfes anzuregen, wenn sich der Prothesenträger länger in einer passiven, beispielsweise sitzenden, Stellung befindet.

Durch US 5,443,525 ist ein Liner bekannt, der zur Aufnahme myoelektrischer Elektroden vorgesehen ist. Hierzu wird in ein Fenster des Prothesenschafts ein nicht metallisches, flexibles und weiches flächiges Polster eingeklebt, in dem sich eine große Anzahl von diskreten leitenden Elektroden befindet. Der Liner besteht vorzugsweise aus Silikon, einem nicht leitenden flexiblen Kunststoff. Die Elektroden können aus einer Mischung aus Silikon und Kohlenstoff oder aus Silikon und Silber gebildet sein, wobei die Elektroden jeweils von nicht leitendem Silikon um-geben sind. Die Elektrodenanordnung ist über das Polster somit auf die Innenseite des Liners geklebt und ist durch das Fenster des Liners zugänglich, sodass die von den Elektroden abgenommenen myoelektrischen Signale durch das Fenster nach außen zur Auswertung bzw. Steuerung geleitet werden können. Diese Anordnung ist aufwendig herzustellen und weist einen begrenzten Tragekomfort auf. Darüber hinaus bedarf das Fenster des Liners eines besonderen Abdichtungsaufwands, wenn der Liner - wie häufig üblich - luftdicht ausgebildet sein muss, um mit Hilfe eines im Innenraum des Liners ausgebildeten Unterdrucks den Liner am Amputationsstumpf zu halten. Der Unterdruck muss dabei vom Liner gegen das Gewicht der bewegten Prothese aufrechterhalten werden.
US 2009/0216339 A1 offenbart einen ähnlichen Liner, bei dem ein zur Verbindung mit einer Elektrode vorgesehener leitender Einsatz in eine entsprechende Öffnung des Liners eingesetzt wird und auf der Außen-seite des Liners am Rand der Öffnung mit einem flanschartigen Rand anliegt. Der Einsatz wird mit dem Liner verklebt. Die Höhe des Einsatzes kann dabei so gewählt werden, dass sie der Dicke des Liners entspricht. Vorgesehen ist aber in erster Linie, dass der Einsatz nach innen über die Innenwandung des Liners hinausragt, um gegen das Gewebe des Amputationsstumpfes zu drücken. Auf der Innenseite des Liners bildet sich zwischen dem Einsatz und der Wandung des Liners ein Luftspalt oder ein mit Klebstoff gefüllter Spalt aus. Auch für diese Konstruktion gelten die oben erwähnten Nachteile, nämlich ein erhöhter Aufwand für die Herstellung einer Luftdichtigkeit der Klebverbindung, die hohen mechanischen Belastungen ausgesetzt sein kann. Bei einem hohen Herstellungsaufwand ist daher die Funktionssicherheit nicht vollständig gewährleistet.
Dieses Problem ergibt sich auch bei der Lösung gemäß DE 20 2006 007 460 U1, bei der eine spezielle Halterung für leitende Elektrodenabschnitte vorgesehen ist, die mit einem flanschartigen Rand an der Innenseite des Liners anliegt.

Aus der US 2010/0114238 A1 ist eine Lineranordnung gemäß des Oberbegriffs von Anspruch 1 bekannt, bei der an der Innenseite des Liners Gelelektroden angeordnet sind. Diese werden beispielsweise mit Silberdraht elektrisch kontaktiert, so dass die von der Elektrode abgenommenen elektrischen Signale ausgewertet werden können oder elektrische Signale über die Elektrode an die Haut abgegeben werden können. Auf der Außenseite des Liners werden die elektrischen Leiter zum proximalen Ende des Liners geführt.

Die DE 10 2007 035 409 A1 beschreibt ein orthopädisches Interface, das mit einem dreidimensionalen Textil ausgestattet. An dessen Innenseite können Elektroden angeordnet sein, die durch elektrische Leiter kontaktiert werden, die durch das Linermaterial hindurchführen. Auf der Außenseite sind folglich lose Kabel angeordnet, die mit entsprechenden Geräten verbunden werden, um Signale auszuwerten oder auf die Elektrode zu senden.

In der nicht vorveröffentlichten DE 10 2010 005 462 A1 ist ein Liner beschrieben, bei dem ein leitender Abschnitt in das Material des Liners integriert ist und mit dem nicht leitenden Material des Liners eine einheitliche, stetig ausgerichtete Innenwand bildet. Das nicht leitende Material des Liners ist dabei vorzugsweise ein Polymerisat, wobei der leitende Abschnitt vor dem Auspolymerisieren des nicht leitenden Materials in das Material eingesetzt werden kann, sodass der leitende Abschnitt beim Auspolymerisieren des Materials des Liners mit diesem zu einem einheitlichen Teil verbunden wird und die stetige, glatte Innenwand ausbildet. Der leitende Abschnitt kann dabei auch aus dem an sich nicht leitenden Material des Liners bestehen, das mit Zusätzen elektrisch leitfähig gemacht worden ist. Der leitende Abschnitt kann gemeinsam mit dem Material des Liners auspolymerisiert werden. Der leitende Abschnitt dient da-bei der elektrischen Verbindung der Hautoberfläche des Amputations-stumpfes mit einer unmittelbar auf der anderen Seite des leitenden Abschnitts angeordneten separaten Elektrode, die elektrische Signale, beispielsweise myoelektrische Signale, von der Haut des Amputationsstumpfs aufnimmt oder alternativ Anregungssignale generiert, die über den leitenden Abschnitt auf die Haut übertragen werden. Hierzu weist der Liner an der Außenseite des elektrisch leitenden Abschnitts beispielsweise eine Aufnahmekammer auf in die eine Elektrode mechanisch eingesetzt werden kann. Die hierfür erforderliche Formung des Liners erfordert zusätzlichen Aufwand.

Die bei Linern für einen Amputationsstumpf bestehenden Probleme bestehen in ähnlicher Form auch für Bandagen, die um ein Körperteil gewickelt werden, wie beispielsweise um eine Extremität, um den Rumpf des Körpers o. dgl.

Der vorliegenden Erfindung liegt die Aufgabe zugrunde, eine Einrichtung der eingangs erwähnten Art so auszubilden, dass sie mit einem einfachen Aufbau für die Übertragung elektrischer Signale geeignet ist.

Zur Lösung dieser Aufgabe ist eine Einrichtung der eingangs erwähnten Art erfindungsgemäß dadurch gekennzeichnet, dass der wenigstens eine Leiter innerhalb des nicht leitenden Materials zu einem distalen Ende der Einrichtung geführt ist.

Die erfindungsgemäße Einrichtung sieht somit einen elektrisch leitenden Abschnitt vor, mit dem das elektrisch leitende Signal von der oder auf die Haut des Körperteils übertragbar ist, ohne hierfür an dieser Stelle die Einrichtung mit einer besonderen Formgebung versehen zu müssen. Vielmehr wird der elektrisch leitende Abschnitt durch das elektrisch nicht leitende Material abgedeckt, sodass der elektrisch leitende Abschnitt nur zur Innenwand hin unabgedeckt bleibt und - abgesehen von der Innenwand - allseitig von nicht leitendem Material umgeben ist. Der elektrisch leitende Abschnitt ist mit einem Leiter verbunden, der durch das elektrisch nicht leitende Material hindurchführt und so das elektrische Signal an eine Stelle der Einrichtung leitet, wo unproblematisch die Verbindung mit einer Mess- oder Auswertungseinrichtung oder mit einem Anregungsstromgenerator hergestellt werden kann. Der Leiter ist dabei als schmales, längliches Element ausgebildet, das vorzugsweise in einer Axialrichtung, des Liners, angeordnet ist. Die axiale Richtung steht senkrecht auf der Umfangsrichtung des röhren- oder trichterförmigen Liners, der an seinem distalen Ende offen oder geschlossen ausgebildet sein kann. Bevorzugt ist eine am distalen Ende geschlossene Ausbildung des Liners.

Der Leiter kann einstückig mit dem elektrisch leitenden Abschnitt ausgebildet sein, also aus demselben Material wie der elektrisch leitende Abschnitt bestehen und vorzugsweise gemeinsam mit diesem hergestellt worden sein.

In einer bevorzugten Ausführungsform der Erfindung ist der leitende Abschnitt in das nicht leitende Material der Wandung integriert und bildet mit dem nicht leitenden Material der Wandung eine einheitlich und stetig ausgebildete Innenwand.

Der elektrisch leitende Abschnitt kann aber auch an einen in dem nicht leitenden Material geführten separaten Leiter angeschlossen sein, der dann aus einem anderen Material, beispielsweise aus Metall, bestehen kann.

Die erfindungsgemäße Einrichtung ist zur Weiterleitung der elektrischen Signale durch die Wandung hindurch an eine geeignete Stelle an der Außenseite der Wandung ausgebildet, an der die Kontaktierung mit einer Signalverarbeitungseinheit oder einem Signalgenerator, beispielsweise innerhalb der Konstruktion einer Prothese oder Orthese leicht möglich ist. Im Falle eines Messsignals wird dieses somit unausgewertet übertragen. Insbesondere bei Messsignalen kann sich dabei ein Problem bezüglich des Signal-Rausch-Verhältnisses ergeben.

In einer besonders bevorzugten Ausführungsform der Erfindung ist unmittelbar auf dem leitenden Abschnitt eine Verstärkerschaltung angeordnet, die von dem nicht leitenden Material der Wandung abgedeckt ist und mit wenigstens einem Anschluss mit dem leitenden Abschnitt verbunden ist. Wenigstens ein anderer Anschluss der Verstärkerschaltung ist mit dem wenigstens einen Leiter verbunden.

Ein von dem elektrisch leitenden Abschnitt von der Haut des Körperteils abgenommenes elektrisches Messsignal kann dadurch unmittelbar an dem elektrisch leitenden Abschnitt vorverstärkt werden, um so als - im Übrigen weiterhin unverarbeitetes - elektrisches Signal mit einem verbesserten Signal-Rausch-Verhältnis über den Leiter übertragen zu werden. Die Verstärkerschaltung ist dabei als integrierte Schaltung ausgebildet und unmittelbar auf dem elektrisch leitenden Abschnitt so angeordnet, dass ein Anschluss der Verstärkerschaltung einen elektrischen Kontakt mit dem elektrisch leitenden Abschnitt erhält, um eine elektrische Verbindung zwischen dem Anschluss und dem elektrisch leitenden Abschnitt herzustellen. Die Verstärkerschaltung ist mit einem anderen Anschluss mit dem Leiter verbunden. Dieser Anschluss kann beispielsweise einer Versorgung mit elektrischem Strom dienen, wobei je nach Anwendungsfall die Verbindung mit einer elektrischen Spannungsquelle oder einer elektrischen Stromquelle herstellbar ist.

In einer derartigen Anordnung kann das elektrische Signal als Messsignal beispielsweise drahtlos auf einen Empfänger übertragen werden. Alternativ ist es möglich, den das verstärkte Messsignal führenden Anschluss der Verstärkerschaltung mit dem Leiter zu verbinden, um so ein vorverstärktes Messsignal über den Leiter zu einer Auswertungsschaltung zu übertragen. Obwohl auch eine drahtlose Stromversorgung, beispielsweise induktiv, möglich ist, wird es in dieser Ausführungsform vorteilhaft sein, einen zweiten Leiter für die Zuführung der elektrischen Energie vorzusehen, der dann mit dem entsprechenden Anschluss der Verstärkerschaltung verbunden ist.

Um für die Zuführung einer Versorgungsspannung nicht zwei in dem nicht leitenden Material der Wandung verlegte Leiter zu benötigen, kann vorgesehen werden, dass die Wandung eine von dem elektrisch leitenden Abschnitt isoliert angeordnete elektrisch leitende Schicht aufweist, die mit einem Anschluss der Stromversorgung verbindbar ausgebildet ist. Vorzugsweise ist mit dieser elektrisch leitenden Schicht der Wandung, die sich auf der Außenseite oder auf der Innenseite des nicht leitenden Materials der Wandung befinden kann, ein Masseanschluss bzw. ein neutraler Leiter der Spannungsversorgung verbunden. Dabei kann eine elektrische Verbindung zu einem entsprechenden Anschluss der Verstärkerschaltung hergestellt werden. Die auf der Außenseite befindliche leitende Schicht bewirkt ferner eine Absicherung der Signalleitung gegen Einstrahlung von Störsignalen.

Befindet sich die leitende Schicht auf der Innenseite der Wandung, sodass sie einen Teil der Innenwand bildet, muss im Bereich des elektrisch leitenden Abschnitts natürlich für eine umgebende Isolierung gesorgt werden, damit ein Messsignal oder Anregungssignal relativ zum Massepotential übertragbar ist und nicht unmittelbar nach Masse abgeleitet wird. In diesem Fall wird sichergestellt, dass die Hautoberfläche des Körperteils außerhalb des Messpunktes auf einem definierten elektrischen Potential liegt, das durch die mit diesem Potential verbundene elektrisch leitende Schicht der Wandung bestimmt wird.

Die Verstärkerschaltung weist vorzugsweise wenigstens einen Transistor, insbesondere einen Feldeffekttransistor auf und ist vorzugsweise durch einen Transistor, insbesondere einen Feldeffekttransistor mit seinen Anschlüssen gebildet. Der Feldeffekttransistor kann dabei in üblichen Verstärkerkonfigurationen geschaltet sein, um je nach Anwendungsfall beispielsweise eine Spannungsverstärkung oder einen Strom-verstärkung zu bewirken.

Durch den Einsatz der Verstärkerschaltung, beispielsweise zur Vorverstärkung eines Messsignals oder zur Impedanzwandlung, wird eine wesentliche Verbesserung einer Signalableitung erzielt, ohne dass hierfür ein besonderer Verdrahtungsaufwand erforderlich wäre.

Vorstehend ist ein einziger leitender Abschnitt einer Einrichtung beschrieben worden. Für den Fachmann ist klar, dass insbesondere für die Abnahme myoelektrischer Signale eine Einrichtung, insbesondere ein Liner, mit mehreren leitenden Abschnitten versehen werden sollte. Ferner ist es möglich, die erfindungsgemäße Anordnung auch für eine Einrichtung, insbesondere Liner, zu verwirklichen, der in bestimmten Bereichen mehrere leitende Abschnitte aufweist, um beispielsweise ein Mess- oder Anregungssignal an einer relevanten Stelle des Körperteils abzunehmen oder zu applizieren. Auf diese Weise können Toleranzen bei der Positionierung der Einrichtung auf dem Körperteil ausgeglichen werden, weil die relevante Stelle des Körperteils nur in einem gewissen Bereich liegen muss und nicht zwingend mit einem einzigen leitenden Abschnitt zusammenfallen muss.

In einer bevorzugten Ausführungsform ist das nichtleitende Material der Wandung ein hydrophobes Material. Alternativ oder zusätzlich dazu besteht der leitende Abschnitt aus einem hydrophilen Material. Dabei können beide Merkmale auch dadurch erreicht werden, dass ein Material mit einer hydrophoben bzw. einer hydrophilen Beschichtung versehen ist. In diesem Fall ist das nichtleitende Material der Wandung mit einer hydrophoben Beschichtung versehen. Der leitende Abschnitt kann mit einer hydrophilen Beschichtung versehen werden.

Um einen möglichst guten elektrischen Kontakt zwischen der Haut des Trägers der Einrichtung und dem leitenden Abschnitt herzustellen, ist es vorteilhaft, wenn sich dazwischen ein Feuchtigkeitsfilm befindet oder der leitende Abschnitt befeuchtet ist. Dies kann mit einem hydrophilen, also wasserfreundlichen, Material einfach erreicht werden. Wird zusätzlich der verbleibende Rest der Innenwand der Einrichtung aus einem hydrophoben Material hergestellt oder mit einer hydrophoben Beschichtung versehen, bleibt dieser Bereich aufgrund der wasserabweisenden Wirkung des Materials trocken. Dies führt zu einer verbesserten Hautverträglichkeit im angelegten Zustand.

Insbesondere wird durch die hydrophobe und hydrophile Eigenschaft der entsprechenden Flächen an der Liner-Innenseite erreicht, dass zumindest nahezu keine Restflüssigkeit an den hydrophoben Anteilen vorhanden ist, so dass die Gefahr von Kurzschlüssen und ungewollten Übertragungen elektrischer Signale verringert oder sogar vollständig beseitigt wird. Dadurch wird eine sauberere Signalübertragung möglich.

Natürlich können aktive und passive Elektroden, also mit integriertem oder separatem Verstärkerelement, verwendet werden.

Eine entsprechende Einrichtung lässt sich besonders einfach reinigen und anlegen. So ist es beispielsweise möglich, die Innenwand der Einrichtung zunächst mit Wasser zu benetzen, beispielsweise die Einrichtung mit Wasser zu füllen. Anschließend wird das Wasser von der Einrichtung entfernt. Dies geschieht beispielsweise durch Auskippen des Wassers aus der Einrichtung. Das hydrophobe, wasserabweisende Material der Innenwandung ist in diesem Fall nahezu vollständig trocken, so dass hier ein angenehmes Tragegefühl und eine hygienische Anwendung erreicht wird. Das hydrophile, wasseranziehende Material des leitenden Bereichs hingegen bleibt befeuchtet und sorgt so für einen guten elektrischen Kontakt.

Die Hydrophobie von Materialien und deren Oberflächen kann beispielsweise durch den Kontaktwinkel angegeben werden. Je größer der Kontaktwinkel ist, desto hydrophober ist die Oberfläche. Dabei werden Oberflächen mit einem Kontaktwinkel von weniger als 90° als hydrophil und Oberflächen mit eine Kontaktwinkel von mehr als 90° als hydrophob bezeichnet.

Die Erfindung soll im Folgenden anhand von in der Zeichnung dargestellten Ausführungsbeispielen näher erläutert werden. Es zeigen:
- Figur 1: eine schematische Darstellung eines Liners mit mehreren elektrisch leitenden Abschnitten, die mit einem mehrpoligen Stecker am distalen Ende des Liners über Leiter verbunden sind, sodass eine Verbindung zu einer Auswertungs- oder Steuerungsschaltung hergestellt ist;
- Figur 2: eine Darstellung gemäß Figur 1 für eine Ausführungsform, bei der der elektrisch leitende Abschnitt mit einem separaten Leiter verbunden ist;
- Figur 3: eine Darstellung gemäß Figur 2, bei der auf dem elektrisch leitenden Abschnitt eine Verstärkerschaltung angeordnet ist, die mit dem Leiter verbunden ist;
- Figur 4: eine Darstellung gemäß Figur 3, bei der die Verstärkerschaltung mit einem weiteren Anschluss mit einer elektrisch leitenden Schicht auf der Außenseite des Liners verbunden ist;
- Figur 5: eine Anordnung gemäß Figur 3, bei der eine elektrisch leitende Schicht auf der Innenseite der Wandung, jedoch isoliert von dem elektrisch leitenden Abschnitt angeordnet ist und ein Anschluss der Verstärkerschaltung mit dieser Schicht verbunden ist;
- Figur 6: eine Darstellung einer Wandung des Liners, die überwiegend aus einem leitenden Material besteht, wobei der leitende Abschnitt von einem elektrisch nicht leitenden Material abgedeckt wird und der mit der Verstärkerschaltung verbundene Leiter innerhalb von nicht leitendem Material geführt ist;
- Figur 7: eine Variante der Anordnung gemäß Figur 6, bei der das nicht leitende Material der Wandung eine leitende Außenschicht von einer leitenden Innenschicht der Wandung trennt;
- Figur 8: eine schematische elektrische Verstärkerschaltung mit einem mit dem elektrisch leitenden Abschnitt verbundenen Eingangsanschluss, ein Ausgangsanschluss sowie zwei Anschlüsse für eine Spannungsversorgung;
- Figur 9: eine Darstellung gemäß Figur 8 für einen Feldeffekttransistor;
- Figur 10: eine Darstellung gemäß Figur 9 für eine Schaltungsvariante, bei der ein Potential der Versorgungsspannung über eine elektrisch leitende Schicht der Wandung zugeführt wird;
- Figur 11: einen schematischen Schnitt durch eine Wandung eines Liners mit einem elektrisch leitenden Abschnitt, der einstückig mit einem elektrischen Leiter ausgebildet ist.

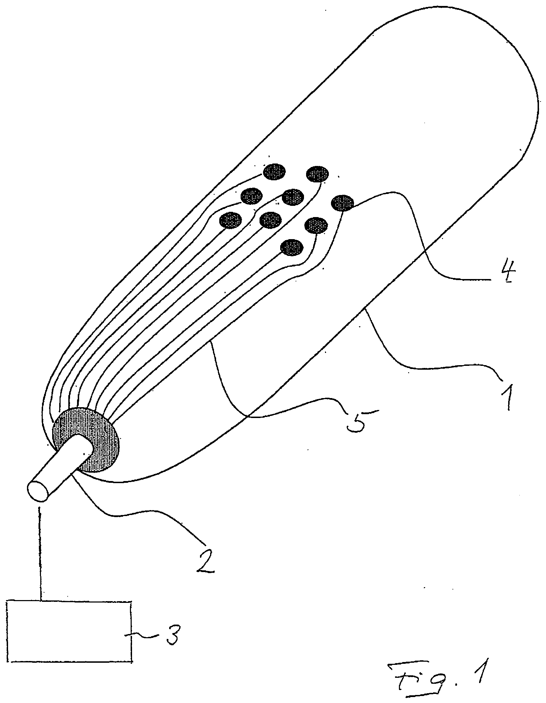

Figur 1 zeigt als Beispiel für eine erfindungsgemäße Einrichtung eine schematische Darstellung eines Liners 1, wie er für eine Bein- oder Armprothese verwendbar ist. Der Liner 1 ist zum Aufziehen auf einen Amputationsstumpf ausgebildet, weist einen hohlen Innenraum auf. In Anpassung an den Amputationsstumpf kann der hülsenförmige Liner zum distalen Ende hin leicht konisch verlaufen. In dem dargestellten Ausführungsbeispiel ist der Liner 1 am distalen Ende geschlossen und hier mit einem Steckeransatz 2 ausgebildet, mit dem der Liner mechanisch mit einer Prothese verbindbar. Gleichzeitig dient der Steckeransatz 2 als elektrisches Verbindungsmittel für eine Auswertungs- oder Steuerungs-schaltung 3. Der Liner dient zur Abpolsterung des Amputationsstumpfes gegenüber einem (nicht dargestellten) Prothesenschaft, mit dem die Prothese am Amputationsstumpf befestigt wird. Zur Verbesserung des Sitzes im Prothesenschaft kann zwischen dem Prothesenschaft und dem Liner 1 und/oder zwischen dem Liner 1 und dem Amputationsstumpf ein Unterdruck angelegt werden, mit dem die jeweilige Haftung verbessert wird.

In Figur 1 ist schematisch dargestellt, dass die Wandung des Liners 1 in einem Bereich mit mehreren elektrisch leitenden Abschnitten 4 versehen ist, die über jeweils einen Leiter 5 mit dem Steckeransatz 2 verbunden sind.

Aus Übersichtsgründen ist nur ein Bereich mit elektrisch leitenden Abschnitten 4 dargestellt. Es versteht sich, dass der Liner auch mehrere Bereiche mit elektrisch leitenden Abschnitten 4 aufweisen kann. Für genau positionierbare Liner 1 ist es ferner denkbar, dass der Liner auch nur einen elektrisch leitenden Abschnitt 4 bzw. in jedem interessierenden Bereich nur einen elektrisch leitenden Abschnitt 4 aufweist.

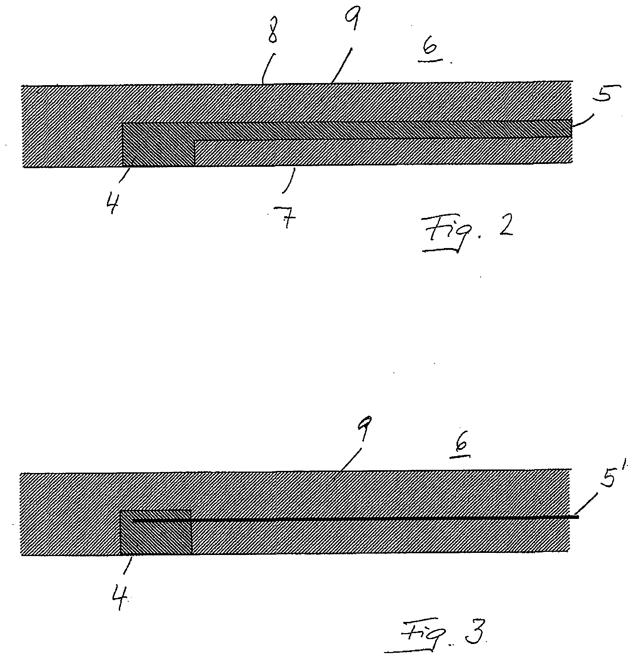

Figur 2 zeigt in einem ersten Ausführungsbeispiel eine Wandung 6 des Liners 1, die eine Innenwand 7 und eine Außenseite 8 aufweist. Die Wandung 6 besteht hierbei aus einem einheitlich nicht leitenden Material 9, das somit die Innenwand 7 und die Außenseite 8 bildet. Auf der Innenwand 7 wird das elektrisch nicht leitende Material 9 durch einen elektrisch leitenden Abschnitt 4 unterbrochen, der in dem dargestellten Ausführungsbeispiel in das nicht leitende Material die einheitliche und stetig ausgebildete Innenwand 7 bildet. In dem dargestellten Ausführungsbeispiel ist der elektrisch leitende Abschnitt 4 - mit Ausnahme der Innenwand 7 - allseitig von dem elektrisch nicht leitenden Material 9 um-geben. Er ist einstückig mit dem innerhalb des nicht leitenden Materials 9 geführten Leiter 5 ausgebildet, sodass elektrische Signale über den elektrisch leitenden Abschnitt 4 und den Leiter 5, beispielsweise an das distale Ende des Liners 1 zu einem Steckeransatz 2 geführt werden können, wie dies an dem in Figur 1 dargestellten Ausführungsbeispiel erkennbar ist.

Bei dem in Figur 3 dargestellten Ausführungsbeispiel ist der elektrisch leitende Abschnitt 4 mit einem separaten Leiter 5' verbunden, der durch das elektrisch nicht leitende Material 9 der Wandung 6 hindurch verläuft, beispielsweise ebenfalls zu einem distalen Ende des Liners 1. Der Leiter 5' kann dabei als metallischer Leiter, beispielsweise Leiterdraht, ausgebildet sein, der bei der Herstellung der Wandung 6 des Liners 1 beispielsweise durch Gießen in die Wandung 6 durch Einlegen in eine entsprechende Gießform eingebracht wird. Die Verbindung des Leiters 5' mit dem als Block ausgeführten elektrisch leitenden Abschnitt 4 kann beispielsweise im noch nicht auspolymerisierten Zustand des elektrisch leitenden Abschnitts erfolgen. Der vorzugsweise aus einem Polymermaterial gebildete elektrisch leitende Abschnitt kann dabei vor oder gleichzeitig mit dem elektrisch nicht leitenden Material 9 der Wandung auspolymerisiert werden.

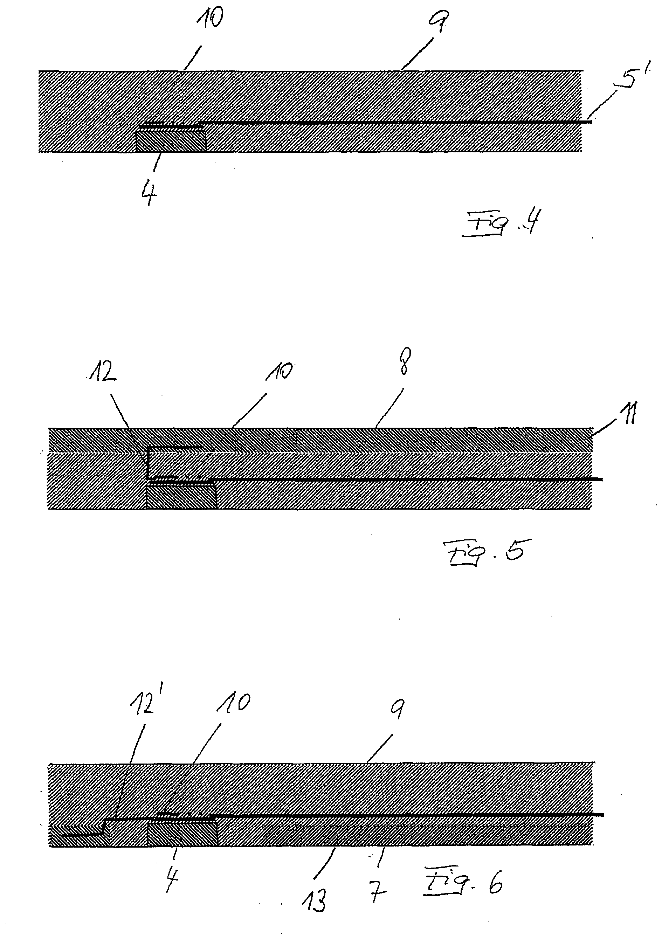

Ein bevorzugtes nicht leitendes Material 9 der Wandung 6 ist Silikon oder Polyurethan. Figur 4 verdeutlicht, dass auf den elektrisch leitenden Abschnitt 4 eine Verstärkerschaltung aufgebracht ist, mit der die von dem elektrisch leitenden Abschnitt 4 aufgenommenen elektrischen Signale vorverstärkt werden können, bevor sie über den Leiter 5', beispielsweise zum Steckeransatz 2, abgeleitet werden. Auch hier ist der elektrisch leitende Abschnitt 4 - zusammen mit der Verstärkerschaltung - allseitig - mit Ausnahme auf der Innenwand 7 - von dem nicht leitenden Material 9 umgeben.

Bei dem in Figur 5 dargestellten Ausführungsbeispiel ist die Wandung auf der Außenseite 8 vollflächig mit einer leitenden Schicht 11 versehen. Diese leitende Schicht ist zweckmäßigerweise mit einem Anschluss einer Spannungsquelle verbunden. Daher kann mit Hilfe der leitenden Schicht 11 in einfacher Weise eine Verbindung 12 der leitenden Schicht 11 zu einem Anschluss der Verstärkerschaltung hergestellt werden, wie dies in Figur 5 verdeutlicht ist.

Bei der in Figur 6 dargestellten Variante befindet sich eine leitende Schicht 13 auf der Innenseite des nicht leitenden Materials 9, sodass die leitende Schicht 13 überwiegend die Innenwand 7 des Liners ausbildet. Die leitende Schicht 13 bildet mit dem leitenden Abschnitt 4 eine fluchtende Innenwand 7 aus, wobei zwischen der leitenden Schicht 13 und dem elektrisch leitenden Abschnitt 4 zur Innenwand 7 hin das nicht leitende Material 9 angeordnet ist, sodass der elektrisch leitende Abschnitt 4 wiederum allseitig - bis auf die Innenwand 7 von nicht leitendem Material 9 umgeben ist. Auch in diesem Ausführungsbeispiel wird ein Anschluss der Verstärkerschaltung 2 über eine Verbindung 12' mit der elektrisch leitenden Schicht 13 verbunden.

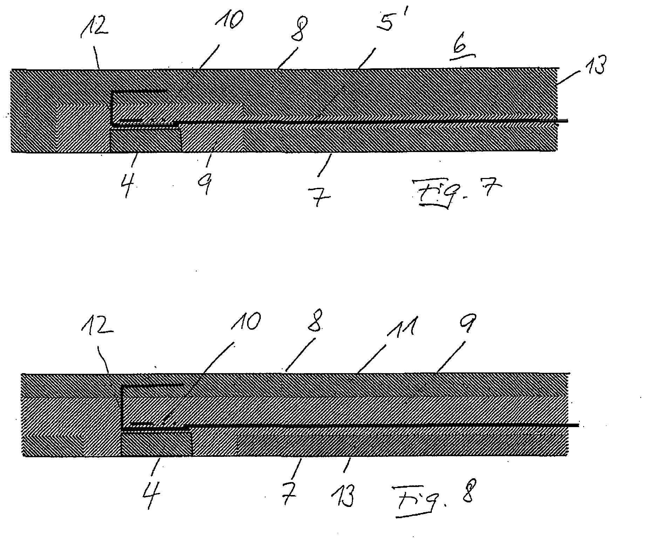

Die in Figur 7 dargestellte Variante zeigt eine Wandung 6 des Liners, die überwiegend aus einer leitenden Schicht 13 gebildet ist, sodass die Außenseite 8 vollständig und die Innenwand 7 überwiegend durch die leitende Schicht gebildet werden. Das nicht leitende Material 9 umgibt allseitig - mit Ausnahme auf der Innenwand 7 - den elektrisch leitenden Abschnitt 4 und die auf ihm angeordnete Verstärkerschaltung 10 und den Leiter 5'.

Bei der in Figur 8 dargestellten Variante besteht die Wandung 6 aus einer leitenden Schicht 11 auf der Außenseite 8, einer elektrisch leitenden Schicht 13 auf der Innenwand 7 und einer zwischen den beiden elektrisch leitenden Schichten angebrachten Zwischenschicht aus dem nicht leitenden Material 9 - wie dargestellt - den elektrisch leitenden Abschnitt 4 mit der darauf angeordneten Verstärkerschaltung 10 allseitig - mit Ausnahme auf der Innenwand 7 - umgibt.

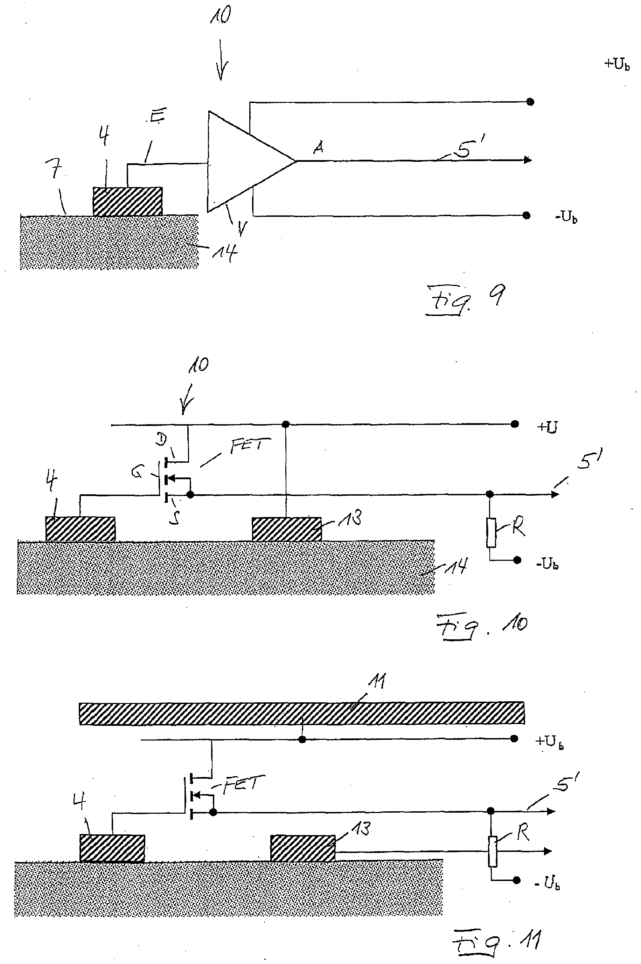

Es wird somit deutlich, dass das nicht leitende Material 9 der Wandung jedenfalls den elektrisch leitenden Abschnitt 4 allseitig abdeckt, sodass dieser nur noch zur Innenwand 7 unabgedeckt bleibt. Darüber hinaus umgibt das nicht leitende Material 9 die Leiter 5, 5' allseitig, um für eine sichere Signalleitung zu sorgen. Das nicht leitende Material bildet bevorzugt eine sich über die gesamte Wandung 6 des Liners erstreckende Schicht aus, kann aber auch, wie dies in Figur 7 verdeutlicht ist, nur stellenweise innerhalb der Wandung 6 des Liners 1 ausgebildet sein. In den Figuren 9 bis 11 sind schematisch mögliche elektrische Verschaltungen für die Verstärkerschaltung 10 dargestellt.

Figur 9 zeigt schematisch den elektrisch leitenden Abschnitt 4, der mit seiner Oberfläche auf der Innenwand 7 auf der Haut 14 eines Amputationsstumpfs aufliegt und über die Haut 14 übertragene elektrische Signale aufnimmt. Das elektrische Signal gelangt über eine Verbindung der Verstärkerschaltung 10 zu einem ersten Anschluss E eines Verstärkers V der Verstärkerschaltung 10. Das verstärkte Signal gelangt über einen Anschluss A auf den Leiter 5, um als verstärktes Signal abgeleitet zu werden. Der Verstärker V ist ferner mit Versorgungsleitungen +U_{b} und -U_{b} verbunden, über die die Betriebsspannung für den Verstärker V zugeführt wird. Figur 9 zeigt somit einen monopolaren Verstärkerbaustein mit Spannungsversorgung.

Figur 10 zeigt eine Verstärkerschaltung 10, die durch einen Feldeffekt-transistor FET gebildet ist. Dieser ist mit einem Anschluss D mit der Versorgungsleitung +U_{b} und mit einem Anschluss S über einen Widerstand R mit der Versorgungsspannung -U_{b} verbunden. Ein weiterer Anschluss G ist direkt mit dem elektrisch leitenden Abschnitt 4 verbunden, sodass diesem Anschluss G elektrische Messsignale von der Haut 14 über den elektrisch leitenden Abschnitt 4 zugeführt werden. Der Feldeffekttransistor FET ist dabei in einer Source-Folger-Schaltung verschaltet, in der keine Spannungsverstärkung, sondern eine Stromverstärkung stattfindet. Sie wirkt als Impedanzwandler und führt zu einer niedrigen Ausgangsimpedanz. Der Verbindungspunkt zwischen dem Anschluss S und dem Widerstand R bildet den Ausgang der Verstärkerschaltung 10 und ist an den Leiter 5' angeschlossen.

Figur 10 verdeutlicht noch, dass ein Pol der Versorgungsspannung (+U_{b}) mit der elektrisch leitenden Schicht 13 verbunden ist, die auf der Haut 14 auftritt, sodass die in Figur 10 dargestellte Schaltung mit den in den Figuren 6 und 7 dargestellten Ausführungsformen realisierbar ist.

Die in Figur 11 dargestellte Schaltung stellt ebenfalls wieder eine Source-Folger-Schaltung eines FET dar, die mit dem Ausführungsbeispiel gemäß Figur 8 realisierbar ist, also mit einer elektrisch leitenden Schicht 11 auf der Außenseite der Wandung 6 und einer elektrisch leitenden Schicht 13 auf der Innenwand 7. Die elektrisch leitende Schicht 13 auf der Innenwand 7 kann hierbei mit einem eigenen Pol verbunden werden, beispielsweise einem Null- oder Masseleiter.

Da der Widerstand R zur Realisierung der Source-Folger-Schaltung außerhalb des Liners 1, beispielsweise in dem Steckeranschluss 2, realisiert werden kann, entsteht auch bei den Verschaltungen gemäß den Figuren 10 und 11 kein zusätzlicher Verdrahtungsaufwand, da lediglich ein Leiter 5' innerhalb des Liners benötigt wird, um das verstärkte Messsignal abzuleiten.

## Patentansprüche

1. Einrichtung mit einer zum engen Umschließen eines Körperteils ausgebildeten Wandung (6) mit einem elektrisch nicht leitenden Material (9) und einer an das Körperteil angepassten oder sich aufgrund der Elastizität der Wandung anpassenden Form, wobei die Wandung (6) mit einer Innenwand (7) an der Haut (14) des Körperteils zur Anlage kommt und die Innenwand (7) mit wenigstens einem elektrisch leitenden Abschnitt (4) versehen ist, der zur Übertragung elektrischer Signale von oder zu der Haut (14) des Körperteils durch das elektrisch nicht leitende Material (9) der Wandung (6) hindurch angeordnet ist, wobei der elektrisch leitende Abschnitt (4) von dem elektrisch nicht leitenden Material (9) so abgedeckt ist, dass der elektrisch leitende Abschnitt nur zu der Innenwand (7) hin unabgedeckt bleibt und mit wenigstens einem durch das elektrisch nicht leitende Material (9) hindurchführenden Leiter (5, 5') verbunden ist, **dadurch gekennzeichnet, dass** der wenigstens eine Leiter (5, 5') innerhalb des nicht leitenden Materials zu einem distalen Ende der Einrichtung geführt ist.

2. Einrichtung nach Anspruch 1, **dadurch gekennzeichnet, dass** der Leiter (5) einstückig mit dem elektrisch leitenden Abschnitt (4) ausgebildet ist.

3. Einrichtung nach Anspruch 1, **dadurch gekennzeichnet, dass** der elektrisch leitende Abschnitt (4) an einen in dem nicht leitenden Material (9) geführten separaten Leiter (5') angeschlossen ist.

4. Einrichtung nach Anspruch 3, **dadurch gekennzeichnet, dass** der Leiter (5') aus Metall besteht.

5. Einrichtung nach einem der Ansprüche 1 bis 4, **dadurch gekennzeichnet, dass** unmittelbar auf dem leitenden Abschnitt (4) eine Verstärkerschaltung (10) angeordnet ist, die von dem nicht leitenden Material (9) der Wandung (6) abgedeckt ist und mit wenigstens einem Anschluss (G) mit dem leitenden Abschnitt (4) verbunden ist und dass wenigstens ein anderer Anschluss (S) der Verstärkerschaltung (10) mit wenigstens einem Leiter (5') verbunden ist.

6. Einrichtung nach Anspruch 5, **dadurch gekennzeichnet, dass** die Wandung (6) eine von dem elektrisch leitenden Abschnitt (4) isoliert angeordnete elektrisch leitende Schicht (11, 13) aufweist, die mit einem Anschluss einer Stromversorgung verbindbar ausgebildet ist.

7. Einrichtung nach Anspruch 6, **dadurch gekennzeichnet, dass** die elektrisch leitende Schicht (13) zum Anschluss an ein Massepotential der Stromversorgung angeordnet ist.

8. Einrichtung nach einem der Ansprüche 5 bis 7, **dadurch gekennzeichnet, dass** die Verstärkerschaltung (10) wenigstens einen Transistor, insbesondere einen Feldeffekttransistor (FET) aufweist.

9. Einrichtung nach Anspruch 8, **dadurch gekennzeichnet, dass** die Verstärkerschaltung (10) durch einen Transistor, insbesondere einen Feldeffekttransistor (FET) mit seinen Anschlüssen gebildet ist.

10. Einrichtung nach einem der Ansprüche 1 bis 9, **dadurch gekennzeichnet, dass** der leitende Abschnitt (4) in das nicht leitende Material (9) der Wandung (6) integriert ist und mit dem nicht leitenden Material (9) der Wandung (6) eine einheitliche und stetig ausgebildete Innenwand (7) bildet.

11. Einrichtung nach einem der vorstehenden Ansprüche, **dadurch gekennzeichnet, dass** das nichtleitende Material (9) der Wandung (6) ein hydrophobes Material ist.

12. Einrichtung nach einem der vorstehenden Ansprüche, **dadurch gekennzeichnet, dass** der leitende Abschnitt (4) aus einem hydrophilen Material besteht.

## Claims

1. Device having a wall (6), which is designed to tightly enclose a body part, comprising an electrically nonconductive material (9) and a shape which is adapted to the body part or adapts thereto by virtue of the elasticity of the wall, wherein the wall (6) comes to bear on the skin (14) of the body part via an inner face (7) and the inner face (7) is provided with at least one electrically conductive section (4), which is arranged in order to transmit electrical signals from or to the skin (14) of the body part through the electrically nonconductive material (9) of the wall (6), wherein the electrically conductive section (4) is covered by the electrically nonconductive material (9) such that the electrically conductive section (4) only remains uncovered toward the inner face (7), and is connected to at least one conductor (5, 5') passing through the electrically nonconductive material (9), **characterized in that** the at least one conductor (5, 5') is guided toward a distal end of the device inside the nonconductive material.

2. Device according to Claim 1, **characterized in that** the conductor (5) is formed integrally with the electrically conductive section (4).

3. Device according to Claim 1, **characterized in that** the electrically conductive section (4) is connected to a separate conductor (5') laid in the nonconductive material (9).

4. Device according to Claim 3, **characterized in that** the conductor (5') consists of metal.

5. Device according to one of Claims 1 to 4, **characterized in that** an amplifier circuit (10), which is covered by the nonconductive material (9) of the wall (6) and is connected by at least one terminal (G) to the conductive section (4), is arranged directly on the conductive section (4), and **in that** at least one other terminal (S) of the amplifier circuit (10) is connected to at least one conductor (5').

6. Device according to Claim 5, **characterized in that** the wall (6) has an electrically conductive layer (11, 13) which is arranged insulated from the electrically conductive section (4) and is formed so that it can be connected to a terminal of an electricity supply.

7. Device according to Claim 6, **characterized in that** the electrically conductive layer (13) is arranged for connection to a ground potential of the electricity supply.

8. Device according to one of Claims 5 to 7, **characterized in that** the amplifier circuit (10) has at least one transistor, in particular a field-effect transistor (FET).

9. Device according to Claim 8, **characterized in that** the amplifier circuit (10) is formed by a transistor, in particular a field-effect transistor (FET) with its terminals.

10. Device according to one of Claims 1 to 9, **characterized in that** the conductive section (4) is integrated into the nonconductive material (9) of the wall (6) and forms a unitary and continuously formed inner face (7) with the nonconductive material (9) of the wall (6).

11. Device according to one of the preceding claims, **characterized in that** the nonconductive material (9) of the wall (6) is a hydrophobic material.

12. Device according to one of the preceding claims, **characterized in that** the conductive section (4) consists of a hydrophilic material.

## Revendications

1. Dispositif avec une paroi (6) réalisée pour envelopper étroitement une partie du corps, avec un matériau non conducteur de l'électricité (9) et une forme adaptée à la partie du corps ou une forme qui s'adapte en raison de l'élasticité de la paroi, dans lequel la paroi (6) vient en contact avec la peau (14) de la partie du corps avec une paroi intérieure (7) et la paroi intérieure (7) est dotée d'au moins un tronçon conducteur de l'électricité (4), qui est agencé de manière à traverser le matériau non conducteur de l'électricité (9) de la paroi (6) afin de transmettre des signaux électriques depuis la peau (14) de la partie du corps ou vers celle-ci, dans lequel le tronçon conducteur de l'électricité (4) est recouvert par le matériau non conducteur de l'électricité (9) de telle façon que le tronçon conducteur de l'électricité (4) reste découvert uniquement vers la paroi intérieure (7) et est relié à au moins un conducteur (5, 5') qui traverse le matériau non conducteur de l'électricité,
**caractérisé en ce que** ledit au moins un conducteur (5, 5') est guidé à l'intérieur du matériau non conducteur jusqu'à une extrémité distale du dispositif.

2. Dispositif selon la revendication 1, **caractérisé en ce que** le conducteur (5) est réalisé d'une seule pièce avec le tronçon conducteur de l'électricité (4).

3. Dispositif selon la revendication 1, **caractérisé en ce que** le tronçon conducteur de l'électricité (4) est branché à un conducteur séparé (5') guidé dans le matériau non conducteur (9).

4. Dispositif selon la revendication 3, **caractérisé en ce que** le conducteur (5') est en métal.

5. Dispositif selon l'une des revendications 1 à 4, **caractérisé en ce qu'**un circuit amplificateur (10) est agencé directement sur le tronçon conducteur (4), circuit qui est recouvert par le matériau non conducteur (9) de la paroi (6) et qui est relié par au moins un connecteur (G) avec le tronçon conducteur (4), et **en ce qu'**au moins un autre connecteur (S) du circuit amplificateur (10) est relié à au moins un conducteur (5').

6. Dispositif selon la revendication 5, **caractérisé en ce que** la paroi (6) comprend une couche électriquement conductrice (11, 13), agencée isolée vis-à-vis du tronçon électriquement conducteur (4), couche qui est réalisée de manière à pouvoir être reliée avec un connecteur d'une alimentation électrique.

7. Dispositif selon la revendication 6, **caractérisé en ce que** la couche électriquement conductrice (13) est agencée en vue de la connexion à un potentiel de masse de l'alimentation électrique.

8. Dispositif selon l'une des revendications 5 à 7, **caractérisé en ce que** le circuit amplificateur (10) comprend au moins un transistor, en particulier un transistor à effet de champ (FET).

9. Dispositif selon la revendication 8, **caractérisé en ce que** le circuit amplificateur (10) est formé par un transistor, en particulier un transistor à effet de champ (FET) avec ses connecteurs.

10. Dispositif selon l'une des revendications 1 à 9, **caractérisé en ce que** le tronçon conducteur (4) est intégré dans le matériau non conducteur (9) de la paroi (6), et forme, avec le matériau non conducteur (9) de la paroi (6), une paroi intérieure unitaire (7) réalisée de manière continue.

11. Dispositif selon l'une des revendications précédentes, **caractérisé en ce que** le matériau non conducteur (9) de la paroi (6) est un matériau hydrophobe.

12. Dispositif selon l'une des revendications précédentes, **caractérisé en ce que** le tronçon conducteur (4) est en un matériau hydrophile.
